# EUROPÄISCHE PATENTANMELDUNG

(11) **EP 4 686 937 A1**
(43) Veröffentlichungstag der Anmeldung: **04.02.2026**
(21) Anmeldenummer: 24191528.9
(22) Anmeldetag: 29.07.2024
(51) Int. Cl.: G01N 27/38, G01N 33/00, G01N 27/404

(54) **REGENERATIONSVERFAHREN FÜR EINEN ELEKTROCHEMISCHEN SENSOR UND KORRESPONDIERENDE SENSORANORDNUNG**

(71) Anmelder: Testo SE & Co. KGaA, 79822 Titisee-Neustadt (DE)
(72) Erfinder: Steiner, Gregor, 79822 Titisee-Neustad (DE); Dornhof, Johannes, 79199 Kirchzarten (DE)
(74) Vertreter: Mertzlufft-Paufler, Cornelius

(57) **Zusammenfassung**

Regenerationsverfahren für einen elektrochemischen Sensor (1), wobei der Sensor (1) wenigstens zwei Elektroden (2) umfasst, wobei die wenigstens zwei Elektroden (2) in einem Normalbetrieb des Sensors (1) in einer ersten Beschaltung betrieben werden. Das Regenerationsverfahren unterscheidet sich vom Normalbetrieb durch eine geänderte Beschaltung der wenigstens zwei Elektroden (2).

## Beschreibung

Die Erfindung betrifft ein Regenerationsverfahren für einen elektrochemischen Sensor, wobei der Sensor wenigstens zwei Elektroden umfasst, wobei die wenigstens zwei Elektroden in einem Normalbetrieb des Sensors in einer ersten Beschaltung betrieben werden.

Die Erfindung betrifft weiter eine Sensoranordnung mit wenigstens einem Sensor, wobei der Sensor wenigstens zwei Elektroden umfasst.

Es hat sich herausgestellt, dass derartige elektrochemische Sensoren, insbesondere elektrochemische Gassensoren, gelegentlich verunreinigt werden, insbesondere durch organische Dämpfe oder organische Mittel, die bei Reinigungsvorgängen eingesetzt werden. Durch diese Verunreinigung (auch Kontamination) kann die korrekte Funktion des Gassensors gestört sein, wodurch beispielsweise hohe Gaskonzentrationen gar nicht mehr oder nicht mehr korrekt erkannt werden. Eine solche Fehlfunktion kann beispielsweise bei Kohlenmonoxid Sensoren (CO-Sensoren) oder Stickstoffmonoxid Sensoren (NO-Sensoren) lebensgefährliche Konsequenzen haben und ist unbedingt zu vermeiden.

Normalerweise ist hier die Handlungsempfehlung, diese Art Substanzen vom Sensor entfernt zu halten, jedoch kommt es in der Praxis vor, dass eine Kontamination mit derartigen Substanzen oder anderen Substanzen auftritt.

Es sind Regenerationsverfahren bekannt, die Temperierungen umfassen, wobei hier eine lange Behandlung des Sensors erforderlich ist, um eine Verunreinigung zu entfernen.

Die Erfindung beschäftigt sich damit, den ursprünglichen Betriebszustand des Sensors möglichst schnell wieder herzustellen.

Diese Aufgabe wird durch ein Regenerationsverfahren mit den Merkmalen des Anspruchs 1 gelöst. Es wird somit zur Lösung der genannten Aufgabe erfindungsgemäß bei einem Regenerationsverfahren der eingangs genannten Art vorgeschlagen, dass das Regenerationsverfahren sich vom Normalbetrieb durch eine geänderte Beschaltung der wenigstens zwei Elektroden unterscheidet. Auf diese Weise können Substanzen, die sich gebildet haben und die durch die Potentialverschiebungen das Reaktionsverhalten des Sensors verändern, beseitigt werden.

Beispielsweise kann die geänderte Beschaltung zu einer Bewirkung von wenigstens einem Stoffumsatz an den wenigstens zwei Elektroden, der sich vom Normalbetrieb unterscheidet, eingerichtet sein. Somit lassen sich beispielsweise Alterungs- und/oder Verunreinigungsprozesse wieder zumindest teilweise oder sogar nahezu vollständig (zum Beispiel bis auf eine fertigungstoleranztypische Abweichung) rückgängig machen.

Das Regenerationsverfahren kann dabei beispielsweise mittels eines Potentiostats realisiert werden. Somit lassen sich auf einfache Weise gewünschte elektrochemische Spannungsverhältnisse einstellen, insbesondere zur Bewirkung eines Stoffumsatzes.

Besonders vorteilhaft ist dabei, dass somit beispielsweise eine Verschiebung eines Arbeitspunktes des Sensors, die beispielsweise aufgrund einer Kontamination einer Elektrode resultiert, erkannt und behoben werden kann. Weiterhin ist besonders vorteilhaft, dass somit das Risiko von Messungenauigkeiten und/oder Messfehlern minimiert und der ursprüngliche Betriebszustand schnell wiederhergestellt werden kann.

Bei einer weiteren vorteilhaften Ausgestaltung kann vorgesehen sein, dass wenigstens eine erste Elektrode zur Detektion einer Kontamination einer zweiten Elektrode geeignet ist. Somit können über auffällige und/oder abnormale Signale an der Hilfselektrode Kontaminationen des Sensors frühzeitig erkannt und Gegenmaßnahmen eingeleitet werden. So ist beispielsweise das Signal der Hilfselektrode eines NO-Sensors bei Anwesenheit von organischen Dämpfen um bis zu mehr als zwei Größenordnungen höher als bei einer NO-Begasung. Zudem hat bei diesem beispielhaft angeführten NO-Sensor die Sensing-Elektrode kein direktes Ansprechverhalten bei Anwesenheit von organischen Dämpfen, so dass diese nicht auf eine Verunreinigung hinweisen kann.

Bei einer weiteren vorteilhaften Ausgestaltung kann vorgesehen sein, dass das Ändern der Beschaltung ein Beaufschlagen der ersten Elektrode mit einem Strom umfasst, insbesondere um einen Stoffumsatz aus der ersten Elektrode zu erzeugen, wobei der Stoffumsatz vorzugsweise im Normalbetrieb an dieser Elektrode nicht stattfindet.

Die mit Strom beaufschlagte Elektrode kann dabei beispielsweise eine Referenzelektrode sein, welche im normalen Betriebsmodus einer elektrochemischen Zelle insbesondere hochohmig in dem drei-Elektroden-Aufbau integriert ist. Als Referenzelektroden bezeichnet man beispielsweise Elektroden mit einem sowohl konstanten als auch sich schnell und reproduzierbaren einstellendem Gleichgewichtspotential. Eingesetzt werden solche Referenzelektroden beispielsweise als Bezugspunkt für die Messung von vorzugsweise relativen Potentialen weiterer Elektroden.

Besonders vorteilhaft ist dabei, dass somit beispielsweise auf der Referenzelektrode gebildete Substanzen, die insbesondere aufgrund einer Potentialverschiebung beispielsweise ein Reaktionsverhalten des Sensors verändern, detektiert und entsprechende Gegenmaßnahmen eingeleitet werden können.

Bei einer weiteren vorteilhaften Ausgestaltung kann es vorgesehen sein, dass die Potentiallage in einem vorgelagerten Selbsttest bestimmt wird. Somit kann der Arbeitspunkt des Sensors bestimmt werden und über den Potentialwert des Reduktionspeaks die wahre oder tatsächliche Potentiallage der Referenzelektrode ermittelt werden.

Bei einer vorteilhaften Ausgestaltung kann vorgesehen sein, dass ein Stromfluss in Abhängigkeit von der angelegten Spannung, insbesondere bei einem Linear Sweep Verfahren, ermittelt wird. Somit kann der Arbeitspunkt des Sensors bestimmt werden und anhand des Peaks die tatsächliche Potentiallage der Referenzelektrode ermittelt werden.

Ein solcher Selbsttest kann beispielsweise mit Hilfe eines sogenannten "Linear Sweep" zur Ermittlung des Stromflusses in Abhängigkeit von der angelegten Spannung durchgeführt werden. Bei dem Linear Sweep handelt es sich um eine (vorzugsweise monotone, beispielsweise monoton steigende oder monoton fallende) Variation der angelegten Spannung, wobei bei der Beschaltung der Elektroden die Gegenelektrode als Sensing-Elektrode und die Hilfselektrode als Gegenelektrode betrieben wird. Mit Hilfe des Selbsttests kann ein Scheitelwert ermittelt werden. Je nach Sensortyp und abhängig von den Materialien, aus welchen die Elektroden gefertigt sind, ist es möglich, statt der Hilfselektrode die Sensing-Elektrode als Gegenelektrode zu verwenden. Beispielsweise ist das Erreichen eines Scheitelwerts als Bedingung nutzbar, um vorzugsweise automatisiert zur nächsten Beschaltung zu wechseln und/oder Diagnosewerte auszugeben. Ein Diagnosewert kann hierbei die Bestätigung eines nicht beeinträchtigen Arbeitspunktes sein, also ein völlig intakter Sensor.

Beispielsweise ist bei einem NO-Sensor die Sensing-Elektrode aus Kohlenstoff, wenn ein selektiver Nachweis von Stickstoffmonoxid (NO) ohne Kohlenmonoxid (CO) gewünscht ist. Die Hilfselektrode und Gegenelektrode können hierbei aus Platin ausgebildet sein.

Bei einer Messung von Kohlenmonoxid (CO) ist die Sensingelektrode ebenso aus Platin ausgebildet, so dass in diesem Fall die Sensing-Elektrode als Gegenelektrode verwendet werden kann.

Die angegebenen Materialien sind allerdings sehr sensorspezifisch und vom Anwendungsgebiet abhängig und sind hier nur beispielhaft zu verstehen.

Mit Hilfe des ermittelten Scheitelwerts kann eine Aussage, bei welcher Spannung der optimale Stoffumsatz stattfindet, getroffen werden. So kann beispielsweise bei darüberliegenden Spannungen noch keine ausreichende Spannung für einen Stoffumsatz erzeugt werden und bei darunterliegenden Spannungen ist der Sensor nicht mehr in der Lage, zusätzlich angebotene Substanzen in kurzer Zeit umzusetzen.

Besonders vorteilhaft ist dabei, dass der Scheitelwert für die Charakterisierung des Sensors verwendet werden kann. Beispielsweise ist der Sensor nicht mehr in der Lage, zusätzliche angebotene Substanzen in kurzer Zeit umzusetzen, wenn der ermittelte Scheitelwert zu nah an dem normalen Arbeitspunkt des Sensors liegt. So reagiert der Sensor beispielsweise bei einer Änderung der Konzentration der nachgewiesenen Substanz oder umgesetzten Substanz nur sehr träge, weil er damit beschäftigt ist, das ihm angebotene Material abzubauen. Ist der ermittelte Scheitelwert sehr weit von dem normalen Arbeitspunkt des Sensors entfernt, besitzt dieser ausreichend Kapazitäten, um vorhandene Substanzen schnell abzubauen und kann somit auf Konzentrationsänderungen sehr schnell reagieren.

Im Normalbetrieb wird z.B. bei einem Sauerstoffsensor der Arbeitswert bei -600 mV gewählt. Zur Vermeidung von Querempfindlichkeiten, beispielsweise von Lachgas, kann der Arbeitswert auch bei einem Wert von -300 mV gewählt werden.

Besonders vorteilhaft ist dabei, dass die Verschiebung des Scheitelwerts darauf hin deutet, dass an anderen Elektroden ein unerwünschter Stoffumsatz und/oder Stoffeintrag stattgefunden hat, der zu einer Veränderung der Potentiallagen führen kann.

Außerdem kann die chemische Zusammensetzung des Sensors sich verändert haben, so dass beispielsweise zwischen bestimmten Elektroden elektrochemische Spannungen auftreten, die dort nicht sein sollten.

Bei einer weiteren vorteilhaften Ausgestaltung kann vorgesehen sein, dass ein Stromfluss in Abhängigkeit von einer angelegten Spannung zwischen zwei der wenigstens drei Elektroden des Sensors, bevorzugt zwischen einer zweiten und einer dritten Elektrode von wenigstens drei Elektroden des Sensors, besonders bevorzugt zwischen einer Gegenelektrode und Hilfselektrode oder zwischen einer Sensing-Elektrode und der Hilfselektrode des Sensors ermittelt wird. Hierbei wird die Gegenelektrode als Sensingelektrode oder Messelektrode und die Hilfselektrode als Gegenelektrode verwendet.

Bei einer weiteren vorteilhaften Ausgestaltung kann vorgesehen sein, dass ein relativer Potentialunterschied zwischen zwei der wenigstens drei Elektroden des Sensors gemessen wird. Beispielsweise kann der Potentialunterschied durch eine Open Circuit Potential - (OCP, Leerlaufpotential) Messung ermittelt werden. Somit kann über eine potentiometrische Bestimmung der Potentialwert zwischen der Referenzelektrode und der Gegenelektrode ermittelt werden. Während der OCP-Messung fließt kein elektrischer Strom, wodurch keine Spannung über den Innenwiderstand der Spannungsquelle abfällt. Die OCP-Messung ist eine passive Messmethode, was bedeutet, dass die Gegenelektrode (die notwendig ist, um Strom durch die Zelle zu leiten) im Schaltkreis des Potentiostaten umgangen wird. In diesem Modus wird nur das Ruhepotential zwischen Referenz- und Sensing-Elektrode gemessen. In der hier beispielhaft beschriebenen Potentialmessung fungiert allerdings die Gegenelektrode als Sensingelektrode.

Bei einer weiteren vorteilhaften Ausgestaltung kann vorgesehen sein, dass ein relativer Potentialunterschied zwischen der ersten Elektrode und einer zweiten oder dritten Elektrode von wenigstens drei Elektroden des Sensors, besonders bevorzugt zwischen einer Referenzelektrode und einer Gegenelektrode des Sensors gemessen wird. Somit können elektrochemische Potentialunterschiede erfasst werden, die im normalen Betrieb des Sensors nicht auftreten sollten. Beispielsweise kann ein Erreichen eines stationären Potentialunterschieds als Bedingung für einen vorzugsweise automatischen Wechsel zu einer nächsten Beschaltung und/oder zu einer Ausgabe von wenigstens einem Diagnosewert verwendet werden.

Bei einer weiteren vorteilhaften Ausgestaltung kann vorgesehen sein, dass wenigstens eine Elektrode amperometrisch bestromt wird. Somit ist es möglich die Dauer der Bestromung und die Höhe der Bestromung zu ermitteln, die nötig ist, um den Sensor zu regenerieren und somit seine Funktionsfähigkeit wiederherzustellen. Als besonders vorteilhaft zur Wiederherstellung der Funktionsfähigkleit hat sich dabei die Bestromung der Referenzelektrode gezeigt. Dies kann beispielsweise zumindest in der geänderten Beschaltung erfolgen.

Insbesondere beim Sauerstoff-Sensor (02-Sensor) hat sich gezeigt, dass die Bestromung der Referenzelektrode die bevorzugte Methode zur direkten und vollständigen Regeneration des Sensors ist. Der O2-Sensor kann infolge einer Vergiftung (Kontamination) in einen falschen Messmodus geraten, bei dem die Potentiallage an der Referenzelektrode so weit verschoben (oder abgesenkt) wird, dass an der Sensing-Elektrode nicht mehr Sauerstoff reduziert wird, sondern Wasserstoff durch Wasserelektrolyse generiert wird. Der an der Meßelektrode entstehende Wasserstoff diffundiert durch den Sensorkörper und lässt das Potential an der Referenzelektrode noch stärker abfallen, so dass die zwischen Referenz- und Gegenelektrode gemessene Spannung um etwa 1 V erhöht wird, also auf etwa 1,5 V .

Dieser fehlerhafte Betrieb kann über zwei Meßgrößen zweifelsfrei erkannt werden. Zum einen wird das Messsignal um ca. 2 Größenordnungen höher, zum Anderen erhöht sich die Potentialdifferenz zwischen der Referenz- und der Gegenelektrode um etwa 1 V. Die Potentialdifferenz zwischen der Referenz- und der Gegenelektrode beträgt somit etwa 1,5 V statt nur 0,5 V.

Zur Regeneration des Sensors wird die Potentialdifferenz zwischen Referenz- und Gegenelektrode als Ausgangsgröße für die anodische Bestromung der Referenzelektrode herangezogen. Wenn nach einer gemessenen Potentialspannung von 1,5 V zwischen Referenz- und der Gegenelektrode die Beschaltung geändert wird und die Gegenelektrode als Sensingelektrode geschaltet, die Referenzelektrode als Sensingelektrode und die Sensingelektrode als Gegenelektrode geschaltet wird, so wird bei einer gewählten Potentialspannung von -600 mV die Potentiallage der Referenzelektrode um ca. 900 mV angehoben. Nach einer derartigen Beschaltung wird der Sensor für eine Zeitdauer von ca. 10 Minuten betrieben. Nach diesem Bestromungsintervall ist der Sauerstoffsensor wieder in seinem ursprünglichen Betriebszustand.

In einem weiteren Ausführungsbeispiel kann ein Erreichen eines bestimmten Stromflusses, der beispielsweise einen vollständigen Stoffumsatz anzeigen kann, als Bedingung für einen vorzugsweise automatischen Wechsel zu einer nächsten Beschaltung und/oder zu einer Ausgabe von wenigstens einem Diagnosewert verwendet werden.

Bei einer weiteren vorteilhaften Ausgestaltung kann vorgesehen sein, dass die Höhe des beaufschlagten Stroms basierend auf dem bestimmten Stromfluss, insbesondere dem bestimmten Spannungswert für den Extremwert des Stromflusses, und/oder dem bestimmten relativen Potentialunterschied, bevorzugt basierend auf einer Addition des Spannungswertes und des Potentialunterschieds bestimmt wird. Besonders vorteilhaft ist dabei, wenn die Referenzelektrode als Sensing-Elektrode, die Gegenelektrode als Referenzelektrode und die Hilfselektrode als Gegenelektrode bestromt wird.

Bei einer weiteren vorteilhaften Ausgestaltung kann vorgesehen sein, dass die Referenzelektrode nicht, wie obenstehend beschrieben, über ein gezieltes Anlegen eines Potentials bestromt wird, sondern über den Betrieb als Gegenelektrode. Besonders vorteilhaft ist dabei, wenn die Referenzelektrode als Gegenelektrode, die Gegenelektrode als Referenzelektrode und die Sensingelektrode unverändert betrieben wird. Über den Strom, der durch die elektrochemische Zelle fließt, lässt sich dann die Ladung und somit das Potential der als Gegenelektrode betriebenen Referenzelektrode in Richtung des Ausgangszustands verschieben.

Besonders vorteilhaft ist dabei, wenn während der Bestromungsvariation die Referenzelektrode bestromt wird. Das beaufschlagte Potential kann aus den Ergebnissen der tatsächlichen Potentiallage der Referenzelektrode und des OCP-Wertes, also der Messung der Potentialspannung zwischen Gegenelektrode und Referenzelektrode berechnet werden.

Bei einer weiteren vorteilhaften Ausgestaltung kann vorgesehen sein, dass zur Kontrolle des Regenerationsverfahrens ein Selbsttest durchgeführt wird. Somit ist es möglich die Wirksamkeit der Regenerationsmaßnahme zu kontrollieren. Besonders vorteilhaft ist, wenn ein derartiges Regenerationsverfahren automatisiert am Sensor durchgeführt werden kann, insbesondere dann, wenn im Normalbetrieb Messwerte erzeugt werden, die außerhalb eines zu erwartenden Bereichs liegen.

Bei einer weiteren vorteilhaften Ausgestaltung kann vorgesehen sein, dass eine erste Elektrode eine Sensing-Elektrode, eine zweite Elektrode eine Hilfselektrode, eine dritte Elektrode eine Gegenelektrode und/oder eine vierte eine Referenzelektrode des Sensors ist. Somit ist es möglich, mit Hilfe einer geänderten Beschaltung der Elektroden ein Regenerationsverfahren zu initiieren, das sich von der Beschaltung der Elektroden von dem Normalbetriebsmodus unterscheidet. Dies kann beispielsweise dadurch geschehen, dass eine Elektrode, die zu einer Spannungsmessung im Normalbetrieb verwendet wird, mit einem Strom beaufschlagt wird, um beispielsweise einen Stoffumsatz zu erzeugen, der üblicherweise im Normalbetrieb an dieser Elektrode nicht stattfindet.

Bei einer weiteren vorteilhaften Ausgestaltung kann vorgesehen sein, dass der Sensor ein Stickstoffmonoxid-Sensor (NO-Sensor) oder ein Kohlenstoffmonoxid-Sensor (CO-Sensor) ist.

Diese Aufgabe wird alternativ oder zusätzlich durch eine Sensoranordnung mit den Merkmalen des nebengeordneten, auf eine Sensoranordnung gerichteten, Anspruchs gelöst. Dabei wird zur Lösung der genannten Aufgabe erfindungsgemäß bei einer Sensoranordnung der eingangs genannten Art vorgeschlagen, dass eine Beschaltungsvorrichtung ausgebildet ist, mit welcher zwischen wenigstens zwei Beschaltungen der wenigstens zwei Elektroden gewechselt werden kann. Es sind somit unterschiedliche Prozesse im elektrochemischen Sensor von außen anregbar. Auf eine mechanische, chemische und/oder thermische Reinigung, die eventuell den Einsatz von chemischen Substanzen erfordert, kann daher verzichtet werden.

Die Beschaltungen können sich beispielsweise durch unterschiedliche Belegungen der Elektroden mit Potentialen und/oder mit Masse und/oder durch unterschiedliche Spannungs- und/oder Strommessungen an den wenigstens zwei Elektroden und/oder durch Einprägung eines Stromes in einer Beschaltung und/oder Anlegen einer Spannung in einer anderen Beschaltung charakterisieren lassen. Somit können die Elektroden einfach von außen in Zustände oder Arbeitsweisen gebracht werden, die sich von einem Normalbetrieb unterscheiden. Es lassen sich somit abweichende, insbesondere umgekehrte, Stoffumsätze in Bezug auf einen, beispielsweise den bereits erwähnten, Normalbetrieb bewirken.

Hierbei kann vorgesehen sein, dass eine erste Beschaltung der wenigstens zwei Beschaltungen für einen Normalbetrieb und/oder eine geänderte Beschaltung für ein Regenerationsverfahren, insbesondere wie zuvor beschrieben und/oder nachfolgend beansprucht, eingerichtet ist/sind.

Bei einer vorteilhaften Ausgestaltung kann vorgesehen sein, dass die Beschaltungseinrichtung zu einem automatischen, vorzugsweise bedingungsgesteuerten, Wechsel zwischen den wenigstens zwei Beschaltungen, insbesondere zwischen wenigstens drei oder wenigstens vier Beschaltungen, eingerichtet ist. Es sind somit komplexere Regenerationsverfahren abbildbar, die eine Abfolge von unterschiedlichen Beschaltungen erfordern.

Beispielsweise können die wenigstens zwei, insbesondere wenigstens drei und/oder wenigstens vier, Beschaltungen aus der Gruppe von
- Normalbetrieb (Mess- und/oder Nachweisbetrieb)
- Bestimmung einer Potentiallage einer Elektrode
- Durchführung eines Linear Sweep
- OCP-Messung
- Amperometrischer Regenerationsbetrieb
- Optional: Überprüfung der Wirksamkeit der durchgeführten Regeneration
ausgewählt sein. Dies ermöglicht es, einzelne oder mehrere der nachstehend beschriebenen Aspekte zu realisieren.

Die erfindungsgemäße Sensoranordnung lässt sich somit vorteilhaft zu einer Realisierung eines erfindungsgemäßen Regenerationsverfahrens nutzen.

Die Erfindung wird nun anhand eines Ausführungsbeispiels näher beschrieben, ist jedoch nicht auf das Ausführungsbeispiel beschränkt. Weitere Ausführungsbeispiele ergeben sich durch die Kombination der Merkmale einzelner oder mehrerer Ansprüche untereinander und/oder mit einzelnen oder mehreren Merkmalen des Ausführungsbeispiels.

Es zeigt:
- Fig. 1:: eine Explosionszeichnung eines erfindungsgemäßen Sensors,
- Fig. 2:: eine schematische Darstellung der Betriebsweise eines elektrochemischen Sensors,
- Fig. 3:: ein Schaltbild des elektrochemischen Sensors im Normalbetrieb,
- Fig. 4:: ein Schaltbild zur Durchführung eines Linear Sweep zur Bestimmung der Potentiallage der Referenzelektrode des elektrochemischen Sensors,
- Fig. 5:: ein Schaltbild zur OCP-Messung zur Bestimmung des Potentialunterschiedes zwischen Gegenelektrode und Referenzelektrode des elektrochemischen Sensors, und
- Fig. 6:: ein Schaltbild zur amperometrischen Regenerationsbestromung des elektrochemischen Sensors.

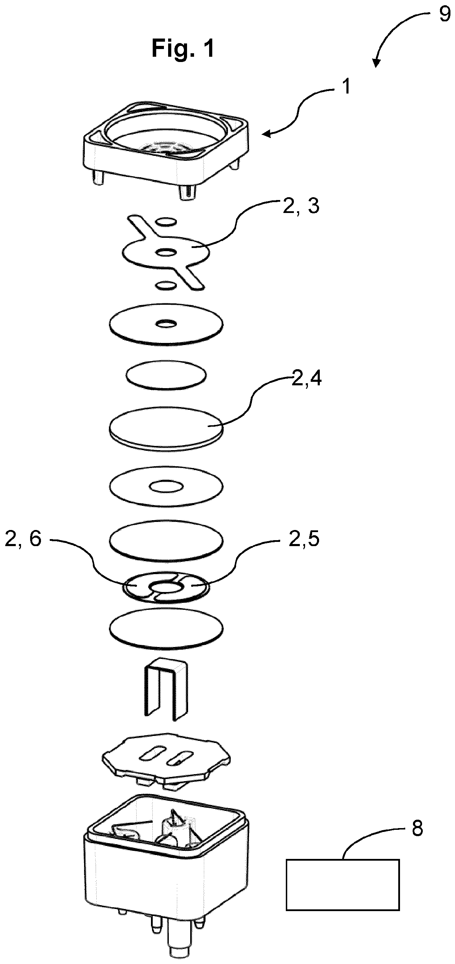

Die Fig. 1 zeigt eine Explosionszeichnung eines Sensors 1, insbesondere eines Gassensors. Solche Sensoren 1 können beispielsweise als Stickstoffmonoxid-Sensoren (NO-Sensoren) oder Kohlenstoffmonoxid-Sensoren (CO-Sensoren) ausgebildet sein.

Der elektrochemische Sensor 1 weist im gezeigten Beispiel von oben nach unten die folgenden Elektroden auf: eine erste Elektrode 2 (Sensing-Elektrode), eine zweite Elektrode 3 (Hilfselektrode), eine dritte Elektrode 4 (Gegenelektrode) und eine vierte Elektrode 5 (Referenzelektrode). Bei den dazwischenliegenden Schichten handelt es sich jeweils um Separatoren oder Abschirmmembranen, die für das erfindungsgemäße Verfahren nicht relevant sind und daher auch nicht weiter beschrieben werden.

Der Sensor 1 ist Teil einer Sensoranordnung 9, die außerdem eine Beschaltungseinrichtung 8 aufweist.

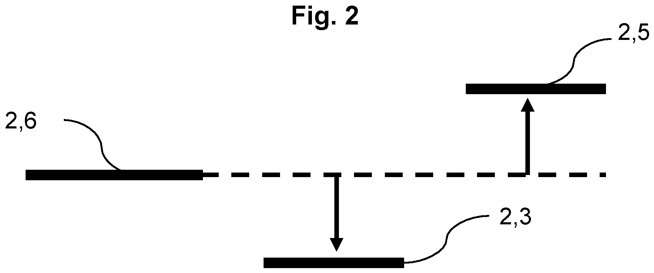

Die Fig. 2 zeigt eine schematische Darstellung der Betriebsweise eines elektrochemischen Sensors. Nach Bestromung wird der Sauerstoff an der Sensing-Elektrode 3 reduziert. Hierbei wird das Potential der Sensing-Elektrode 2 um 600 mV gegenüber der Referenzelektrode 6 abgesenkt. Sobald der Sensor durch diese Bestromung seinen Betrieb aufnimmt, wird durch die Absenkung des Potentials an der Sensing-Elektrode 2 Sauerstoff reduziert. Gleichzeitig muss eine Gegenreaktion stattfinden, um für einen Ladungsausgleich zu sorgen, was bedeutet, dass an der Gegenelektrode 5 eine entsprechende Gegenreaktion stattfindet. Hierbei wird Wasser elektrolytisch/anodisch zerlegt und Sauerstoff wird freigesetzt. Dadurch steigt das Potential der Gegenelektrode 5 gegenüber der Referenzelektrode 6 um einige hundert mV an. Diese Potentialverteilung ist im Normalbetrieb und bei konstanter Bestromung stabil.

Bei Kontamination des Sensors 1 durch organische Substanzen kann es zu einer Vergiftung des elektrochemischen Sensors 1 kommen und infolgedessen zu einer fehlerhaften Messung des elektrochemischen Sensors 1 führen.

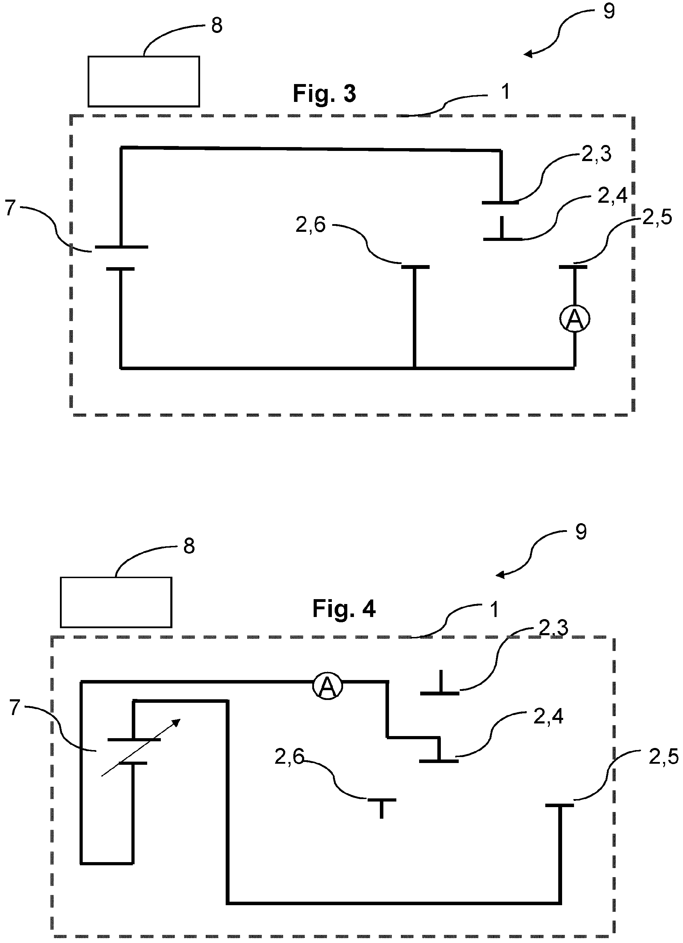

Fig. 3 zeigt ein Schaltbild des elektrochemischen Sensors 1 im Normalbetrieb mit einer Spannungsquelle 7. Wie bereits zu Fig. 2 ausgeführt, wird nach der Bestromung der Sauerstoff an der Sensing-Elektrode 2 reduziert. Zum Ladungsausgleich muss gleichzeitig eine Gegenreaktion stattfinden. Hierbei findet an der Gegenelektrode 5 eine entgegengesetzte Reaktion statt, d.h. Wasser wird elektrolytisch an der Gegenelektrode 5 zerlegt und Sauerstoff freigesetzt.

Die in den Fig. 4 bis Fig. 6 dargestellten Schritte zeigen beispielhaft ein Regenerationsverfahren, um den ursprünglichen Betriebszustand eines elektrochemischen Sensors 1 wieder herzustellen.

Durch eine sich vom Normalbetrieb geänderte Beschaltung der Elektroden ist es möglich, eine beschleunigte Wiederherstellung der Messfähigkeit nach Vergiftung eines elektrochemischen Gassensors 1 durchzuführen.

Die Vorgehensweise wird anhand der folgenden Schritte des Ausführungsbeispiels zur Detektion und Regeneration des Gassensors 1 dargestellt:
Im ersten nicht dargestellten Schritt kann die Hilfselektrode 3 zur Detektion von Kontaminationen des elektrochemischen Sensors 1 eingesetzt werden. Hierzu wird die Beschaltung gegenüber dem Normalbetrieb verändert.

Hierbei können auffällige Signale an der Hilfselektrode 4 Abweichungen zum normalen Betriebszustand des elektrochemischen Sensors 1 anzeigen. Beispielsweise kann das Signal an der Hilfselektrode 4 bei Anwesenheit von organischen Dämpfen um bis zu mehr als zwei Größenordnungen höher als im Normalbetrieb sein, wohingegen die Sensingelektrode 3 kein direktes Ansprechverhalten bei Anwesenheit von organischen Dämpfen zeigt.

Die gemessenen Größen/Verläufe und/oder ein daraus abgeleiteter Wert kann/können als Diagnosewert ausgegeben werden.

Im nächsten Schritt des Regenerationsverfahrens wird die Größe der Bestromung in einem Selbsttest des Sensors 1 ermittelt. Hierzu wird die Beschaltung verändert.

Fig. 4 zeigt die Schaltung, bei welcher mittels der Linear Sweep Methode der Stromfluss in Abhängigkeit von der angelegten Spannung ermittelt wird. Hierbei wird der Arbeitspunkt des Sensors 1 bestimmt. Die Gegenelektrode 5 wird hier als Sensing-Elektrode 3 und die Hilfselektrode 4 als Gegenelektrode 5 bestromt, so dass über den Potentialwert des Reduktionspeaks die tatsächliche Potentiallage der Referenzelektrode 6 bestimmt werden kann.

Die gemessene Größe und/oder ein daraus abgeleiteter Wert kann/können als Diagnosewert ausgegeben werden.

Im weiteren Verlauf des Regenerationsverfahrens wird ein relativer Potentialunterschied zwischen zwei der Elektroden 2 erfasst. Hierzu wird die Beschaltung der Elektroden 2 erneut verändert.

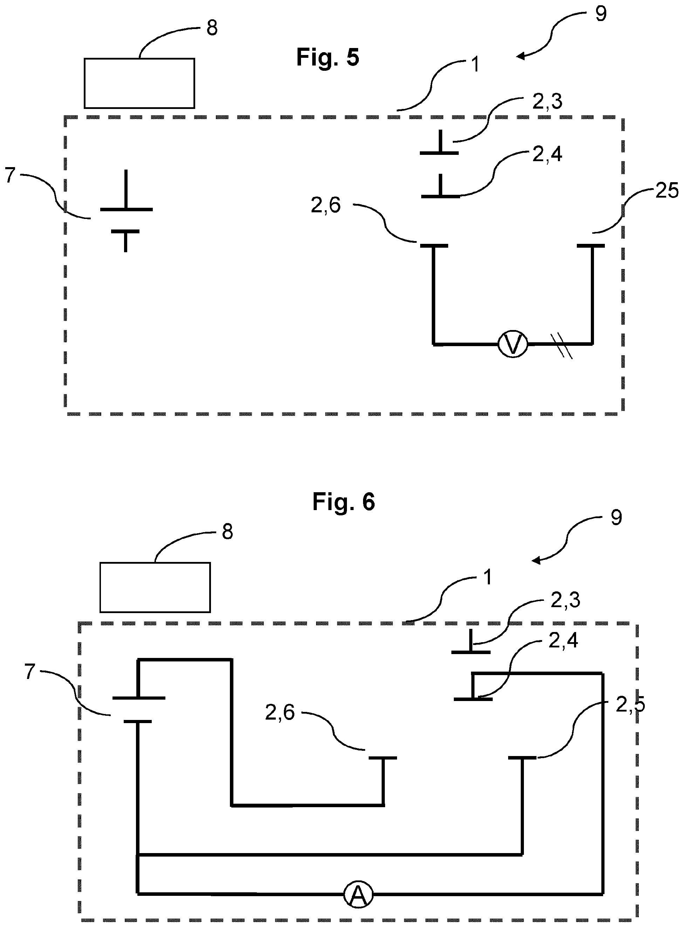

Fig. 5 des erfindungsgemäßen Ausführungsbeispiels zeigt eine Open Circuit Potentialmessung (OCP). Hierbei findet eine Messung ohne Stromfluss statt. Dabei wird der Potentialwert zwischen der Referenzelektrode 6 und Gegenelektrode 5 bestimmt, das heißt der relative Potentialunterschied der beiden Elektroden 2 wird gemessen.

Die gemessene OCP-Größe und/oder ein daraus abgeleiteter Wert kann/können als Diagnosewert ausgegeben werden.

Bei dem nächsten Schritt des Regenerationsverfahrens findet eine amperometrische Messung statt. Hierzu wird die Beschaltung der Elektroden 2 verändert. Wie in Fig. 5 dargestellt, wird die Referenzelektrode 6 bestromt. Das hierzu zu beaufschlagende Potential ergibt sich aus der Addition der Messwerte der vorherigen Schritte, das heißt aus Addition des Peak der Linear Sweep Messung und dem Potential zwischen Gegenelektrode 5 und Referenzelektrode 6.

Die gemessene Größe, beispielsweise eine Zeitspanne bis zu einem Ende der amperometrischen Messung, und/oder ein daraus abgeleiteter Wert kann/können als Diagnosewert ausgegeben werden.

In einer nicht dargestellten Ausführungsform wird zur Kontrolle des Regenerationsverfahrens mittels der Linear Sweep Methode ein Selbsttest durchgeführt, um die Wirksamkeit des Verfahrens zu überprüfen und/oder das Ergebnis der Regeneration zu ermitteln.

Hierbei wird das Regenerationsverfahren so lange initiiert, bis der Stromfluss einen vollständigen Stoffumsatz anzeigt. Im Idealfall soll der Potentialwert der Referenzelektrode 5 bei - 300 ± 50 mV im Vergleich zum Potentialwert vor der Regeneration liegen, so dass wieder Gleichgewichtslage hergestellt ist.

Jedes der vorstehend beschriebenen Schaltbilder stellt eine Beschaltung der wenigstens zwei Elektroden 2 dar. Die Beschaltungsvorrichtung 8 ist in nicht näher gezeigter Weise durch Umschaltvorrichtungen und andere Schalter dazu eingerichtet, zwischen den Beschaltungen auf einzelne oder mehrere Steuerungssignale hin wechseln zu können. Somit lassen sich die beschriebenen oder weitere Abläufe mit automatischen, durch das Erreichen von Bedingungen wie Stationarität von Messwerten, Erreichen von Schwellwerten der Messwerte und/oder Verstreichen von Zeitabschnitten, realisieren.

Die Erfindung betrifft somit ein Regenerationsverfahren für einen elektrochemischen Sensor 1, wobei der Sensor 1 wenigstens zwei Elektroden 2 umfasst, wobei die wenigstens zwei Elektroden 2 in einem Normalbetrieb des Sensors 1 in einer ersten Beschaltung betrieben werden. Das Regenerationsverfahren unterscheidet sich vom Normalbetrieb durch eine geänderte Beschaltung der wenigstens zwei Elektroden 2.

### Bezugszeichenliste

- 1: Sensor
- 2: Elektrode
- 3: Sensing-Elektrode
- 4: Hilfselektrode
- 5: Gegenelektrode
- 6: Referenzelektrode
- 7: Spannungsquelle
- 8: Beschaltungseinrichtung
- 9: Sensoranordnung

## Patentansprüche

1. Regenerationsverfahren für einen elektrochemischen Sensor (1), wobei der Sensor (1) wenigstens zwei Elektroden (2) umfasst, wobei die zwei Elektroden (2) in einem Normalbetrieb des Sensors (1) in einer ersten Beschaltung betrieben werden, **dadurch gekennzeichnet, dass** das Regenerationsverfahren sich vom Normalbetrieb durch eine geänderte Beschaltung der wenigsten zwei Elektroden (2) unterscheidet, insbesondere zum Bewirken von wenigstens einem Stoffumsatz an den wenigstens zwei Elektroden (2), der sich vom Normalbetrieb unterscheidet.

2. Regenerationsverfahren nach Anspruch 1, **dadurch gekennzeichnet, dass** wenigstens eine erste Elektrode (2) zur Detektion einer Kontamination einer zweiten Elektrode (2) geeignet ist.

3. Regenerationsverfahren nach einem der vorangehenden Ansprüche, wobei eine erste Elektrode (2) der wenigstens zwei Elektroden (2) im Normalbetrieb zu einer Spannungsmessung verwendet wird, **dadurch gekennzeichnet, dass** das Ändern der Beschaltung ein Beaufschlagen der ersten Elektrode (2) mit einem Strom umfasst, insbesondere um einen Stoffumsatz an der ersten Elektrode (2) zu erzeugen, wobei der Stoffumsatz vorzugsweise im Normalbetrieb an dieser Elektrode (2) nicht stattfindet.

4. Regenerationsverfahren nach einem der vorangehenden Ansprüche, **dadurch gekennzeichnet, dass** die Höhe des beaufschlagten Stroms durch einen Selbsttest des Sensors (1) bestimmt wird.

5. Regenerationsverfahren nach einem der vorangehenden Ansprüche, **dadurch gekennzeichnet, dass** ein Stromfluss in Abhängigkeit von der angelegten Spannung, insbesondere bei einem Linear Sweep Verfahren, ermittelt wird.

6. Regenerationsverfahren nach einem der vorangehenden Ansprüche, **dadurch gekennzeichnet, dass** ein Stromfluss in Abhängigkeit von einer angelegten Spannung zwischen zwei der wenigstens zwei Elektroden (2) des Sensors (1), bevorzugt zwischen einer zweiten und einer dritten Elektrode (2) von wenigstens drei Elektroden (2) des Sensors (1), besonders bevorzugt zwischen einer Gegenelektrode (5) und einer Hilfselektrode (4) des Sensors oder zwischen einer Sensing-Elektrode (3) und der Hilfselektrode (4) des Sensors (1) ermittelt wird.

7. Regenerationsverfahren nach einem der vorangehenden Ansprüche, **dadurch gekennzeichnet, dass** ein relativer Potentialunterschied zwischen zwei der wenigstens drei Elektroden (2) des Sensors (1) gemessen wird.

8. Regenerationsverfahren nach einem der vorangehenden Ansprüche, **dadurch gekennzeichnet, dass** ein relativer Potentialunterschied zwischen der ersten Elektrode (2) und einer zweiten oder dritten Elektrode (2) von wenigstens drei Elektroden (2) des Sensors gemessen wird, besonders bevorzugt zwischen einer Referenzelektrode (6) und einer Gegenelektrode (5) des Sensors (1).

9. Regenerationsverfahren nach einem der vorangehenden Ansprüche, **dadurch gekennzeichnet, dass**, insbesondere zumindest in der geänderten Beschaltung, wenigstens eine Elektrode (2) amperometrisch bestromt wird.

10. Regenerationsverfahren nach einem der vorangehenden Ansprüche, **dadurch gekennzeichnet, dass** die Höhe des beaufschlagten Stroms basierend auf dem bestimmten Stromfluss, insbesondere dem bestimmten Spannungswert für den Extremwert des Stromflusses und/oder dem bestimmten relativen Potentialunterschied, bevorzugt basierend auf einer Addition des Spannungswertes und des Potentialunterschieds bestimmt wird.

11. Regenerationsverfahren nach einem der vorangehenden Ansprüche, **dadurch gekennzeichnet, dass** zur Kontrolle des Regenerationsverfahrens ein Selbsttest durchgeführt wird.

12. Regenerationsverfahren nach einem der vorangehenden Ansprüche, **dadurch gekennzeichnet, dass** eine erste Elektrode (2) eine Sensing-Elektrode (3), eine zweite Elektrode (2) eine Hilfselektrode (4), eine dritte Elektrode (2) eine Gegenelektrode (5) und/oder eine vierte Elektrode (2) eine Referenzelektrode (6) des Sensors ist.

13. Regenerationsverfahren nach einem der vorangehenden Ansprüche, **dadurch gekennzeichnet, dass** der Sensor (1) ein amperometrischer Gassensor ist, insbesondere ein Stickstoffmonoxid-Sensor (NO-Sensor) und/oder ein Kohlenstoffmonoxid-Sensor (CO-Sensor) oder ein Sauerstoffsensor (O2-Sensor).

14. Sensoranordnung (8) mit wenigstens einem elektrochemischen Sensor (1), wobei der Sensor (1) wenigstens zwei Elektroden (2) umfasst, und einer Beschaltungsvorrichtung (7), mit welcher zwischen wenigstens zwei Beschaltungen der wenigstens zwei Elektroden (2) gewechselt werden kann, insbesondere wobei eine erste Beschaltung der wenigstens zwei Beschaltungen für einen Normalbetrieb und/oder eine geänderte Beschaltung für ein Regenerationsverfahren, insbesondere nach einem der Ansprüche 1 bis 13, eingerichtet ist/sind.

15. Sensoranordnung (8) nach Anspruch 14, **dadurch gekennzeichnet, dass** die Beschaltungseinrichtung (7) zu einem automatischen, vorzugsweise bedingungsgesteuerten, Wechsel zwischen den wenigstens zwei Beschaltungen, insbesondere zwischen wenigstens drei oder wenigstens vier Beschaltungen, eingerichtet ist.
